# EUROPEAN PATENT APPLICATION

(11) **EP 2 716 316 A1**
(43) Date of publication of application: **09.04.2014**
(21) Application number: 12187310.3
(22) Date of filing: 04.10.2012
(51) Int. Cl.: A61M 5/178

(54) **Medicament delivery device with damping mechanism**

(71) Applicant: Sanofi-Aventis Deutschland GmbH, 65929 Frankfurt am Main (DE)
(72) Inventor: The designation of the inventor has not yet been filed

(57) **Abstract**

Described is a medicament delivery device (1) comprising a plunger (5), a damper plunger (8) coupled to the plunger (5) such that translation of the plunger (5) causes translation of the damper plunger (8), and a chamber (7) filled with a shear thickening fluid (F). Translation of the damper plunger (8) displaces the shear thickening fluid (F) within the chamber (7).

## Description

### Technical Field

The invention relates to a medicament delivery device with a damping mechanism.

### Background of the Invention

Administering drugs using drug delivery devices, e.g. automatic injection devices, is a process which is intended to have a repeatable and consistent medicament delivery profile. For example, an injection time should be within a required tolerance and consistent throughout a production line of devices. Injection time, however, is influenced by a number of parameters, such as drug viscosity, siliconisation of the syringe wall, diameter of the syringe, needle diameter, force of a drive spring, friction in the injection device, etc. These parameters are subject to tolerances which may compound and result in large fluctuations in injection time.

Thus, there remains a need for an improved medicament delivery device with a damping mechanism.

### Summary of the Invention

Described is a medicament delivery device with a damping mechanism.

In an exemplary embodiment, a medicament delivery device according to the present invention comprises a plunger, a damper plunger coupled to the plunger such that translation of the plunger causes translation of the damper plunger, and a chamber filled with a shear thickening fluid. Translation of the damper plunger displaces the shear thickening fluid within the chamber.

In an exemplary embodiment, the damper plunger is integrally formed with the plunger.

In an exemplary embodiment, the damper plunger includes a plurality of piston legs arranged in parallel with the plunger. The piston legs each include distal ends disposed in the chamber. The distal ends have a larger cross-section than the piston legs and a smaller cross-section than the chamber providing a fluid gap between the distal ends and the chamber. A position of the distal ends relative to the chamber defines a distal volume and a proximal volume of the fluid. Translation of the piston legs relative to the chamber causes the distal ends to displace the fluid from the distal volume to the proximal volume.

In an exemplary embodiment, the medicament delivery device further comprises a syringe containing a stopper, a medicament and a needle. The plunger is adapted to push the stopper to expel the medicament through the needle.

The exemplary embodiments of the present invention ensure constant injection times and uniform injection speed profiles by compensating fluctuations of parameters, such as drug viscosity, siliconisation of the syringe wall, diameter of the syringe, needle diameter, force of a drive spring, friction in the injection device, etc. The damping mechanism of the present invention leads to a stronger damping at higher velocities and a weaker damping at lower velocities.

Further scope of applicability of the present invention will become apparent from the detailed description given hereinafter. However, it should be understood that the detailed description and specific examples, while indicating preferred embodiments of the invention, are given by way of illustration only, since various changes and modifications within the spirit and scope of the invention will become apparent to those skilled in the art from this detailed description.

### Brief Description of the Drawings

The present invention will become more fully understood from the detailed description given hereinbelow and the accompanying drawings which are given by way of illustration only, and thus, are not limitive of the present invention, and wherein:
- Figure 1: is a perspective view of an exemplary embodiment of a medicament delivery device with a damping mechanism according to the present invention,
- Figure 2: is a side view of an exemplary embodiment of a medicament delivery device with a damping mechanism according to the present invention, and
- Figure 3: is a longitudinal section of an exemplary embodiment of a medicament delivery device with a damping mechanism according to the present invention.

Corresponding parts are marked with the same reference symbols in all figures.

### Detailed Description

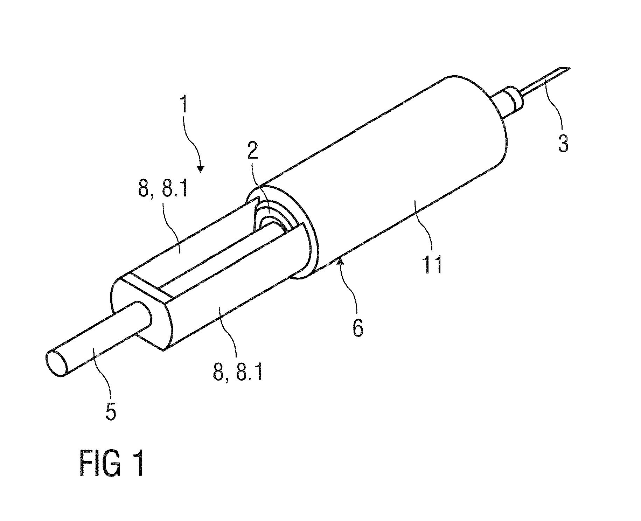

Figure 1 is a perspective view of an exemplary embodiment of a medicament delivery device 1. The medicament delivery device 1 may be an type of device adapted for delivery of a medicament such as, for example, an auto-injector, a pen injector, a syringe, an infusion device, etc.

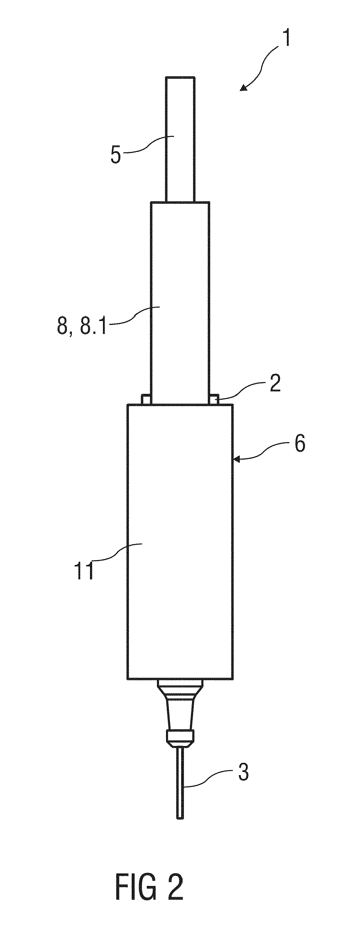

In the exemplary embodiments shown in Figures 1 and 2, the delivery device 1 comprises a syringe 2 with a needle 3, and a plunger 5 operably coupled to a damping mechanism 6. Those of skill in the art will understand that other components, e.g., a drive spring, a case, a trigger buton, etc., may be included in the delivery device 1.

In an exemplary embodiment, the damping mechanism 6 includes a damper plunger 8. The damper plunger 8 may be coupled to or integrally formed wit the plunger 5, such that axial translation of the plunger 5 relative to the delivery device 1 results in corresponding axial translation of the damper plunger 8. The damper plunger 8 may include one or more piston legs 8.1.

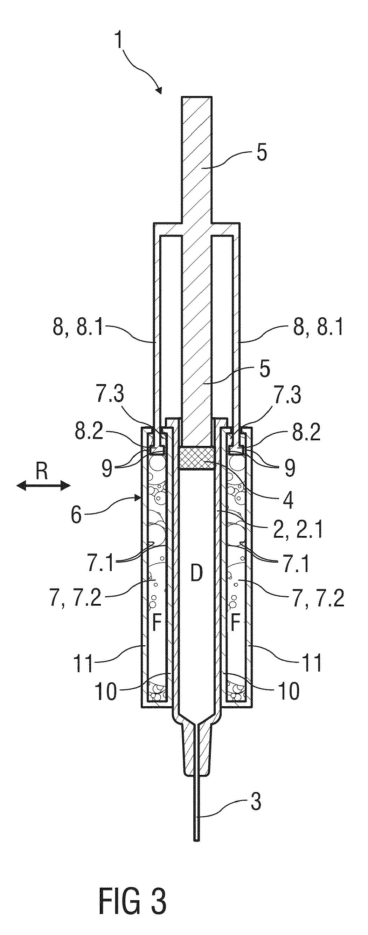

As shown in the exemplary embodiment in Figure 3, the damping mechanism 6 includes the damper plunger 8 which is operably coupled to a chamber 7 having an outer wall 11 and an inner wall 10. The chamber 7 defines a volume 7.1 for containing a fluid F, preferably a dilatant or shear thickening fluid [examples?]. Those of skill in the art will understand that the fluid F may be a liquid and/or a gas. In the exemplary embodiment, the syringe 2 is contained by the chamber 7, e.g., between the inner walls 10.

In an exemplary embodiment, the damper plunger 8 includes two piston legs 8.1 which are integrally formed with and arranged in parallel with the plunger 5. The piston legs 8.1 may extend through a proximal wall of the chamber 7 and into the volume 7.1. Distal ends 8.2 of the piston legs 8.1 may have a larger cross-section than the piston legs 8.1 to retain the distal ends 8.2 in the chamber 7. The cross-section of the chamber 7 may be larger than the cross-section of the distal ends 8.2 such a radial gap 9 is provided between the distal ends 8.2. The fluid F may pass through the radial gap 9 between a distal volume 7.2 and a proximal volume 7.3 of the chamber 7. The fluid F is sealed by the distal, outer and inner walls of the chamber 7 and the presence of the piston legs 8.1 in the apertures in the proximal wall.

In an exemplary embodiment, the syringe 2 is arranged between the inner walls 10. An axial position of the syringe 2 relative to the chamber 7 may be fixed, because finger flanges on a proximal end of the syringe 2 may abut a proximal wall of the chamber 7.

Figure 3 shows an exemplary embodiment of the present invention prior to use. During use, when the plunger 5 translates relative to the chamber 7, the plunger 5 pushes a stopper 4 axially relative to a syringe body 2.1 to expel the medicament D through the needle 3. As plunger 5 translates, the damper plunger 8 translates concurrently. Thus, the piston legs 8.1 translate relative to the chamber 7, and the distal ends 8.2 displace the fluid 7. As the fluid F passes through the gaps 9, the distal volume 7.2 is reduced and the proximal volume 7.3 increases. The dilatant behaviour of the fluid F leads to a stronger damping at higher velocities and a weaker damping at lower velocities. Thus, the velocity profile of the injection is more uniform and the injection time is repeatable.

Those of skill in the art will understand that modifications (additions and/or removals) of various components of the apparatuses, methods and/or systems and embodiments described herein may be made without departing from the full scope and spirit of the present invention, which encompass such modifications and any and all equivalents thereof.

## Claims

1. A medicament delivery device (1) comprising:
a plunger (5);
a damper plunger (8) coupled to the plunger (5) such that translation of the plunger (5) causes translation of the damper plunger (8); and
a chamber (7) filled with a shear thickening fluid (F),
wherein translation of the damper plunger (8) displaces the shear thickening fluid (F) within the chamber (7).

2. The medicament delivery device (1) according to claim 1, wherein the damper plunger (8) is integrally formed with the plunger (5).

3. The medicament delivery device (1) according to any one of the preceding claims, wherein the damper plunger (8) includes a plurality of piston legs (8.1) arranged in parallel with the plunger (5).

4. The medicament delivery device (1) according to claim 3, wherein the piston legs (8.1) each include distal ends (8.2) disposed in the chamber (7).

5. The medicament delivery device (1) according to claim 4, wherein the distal ends (8.2) have a larger cross-section than the piston legs (8.1) and a smaller cross-section than the chamber (7) providing a fluid gap (9) between the distal ends (8.2) and the chamber (7).

6. The medicament delivery device (1) according to claim 5, wherein a position of the distal ends (8.2) relative to the chamber (7) defines a distal volume (7.2) and a proximal volume (7.3) of the fluid (F).

7. The medicament delivery device (1) according to claim 6, wherein translation of the piston legs (8.1) relative to the chamber (7) causes the distal ends (8.2) to displace the fluid (7) from the distal volume (7.2) to the proximal volume (7.3).

8. The medicament delivery device (1) according to any one of the preceding claims, further comprising:
a syringe (2) containing a stopper (4), a medicament (D) and a needle (3), and
wherein the plunger (5) is adapted to push the stopper (4) to expel the medicament (D) through the needle (3).
